# EUROPEAN PATENT SPECIFICATION

(11) **EP 4 221 798 B1**
(45) Date of publication and mention of the grant of the patent: **03.12.2025**
(21) Application number: 21778493.3
(22) Date of filing: 22.09.2021
(51) Int. Cl.: A61M 16/08, A61M 16/06

(54) **EXHAUST ARRANGEMENT FOR PATIENT INTERFACE DEVICE AND PATIENT INTERFACE INCLUDING SAME**
ABGASANORDNUNG FÜR EINE PATIENTENSCHNITTSTELLENVORRICHTUNG UND PATIENTENSCHNITTSTELLE DAMIT
AGENCEMENT D'ÉCHAPPEMENT POUR DISPOSITIF D'INTERFACE PATIENT ET INTERFACE PATIENT LE COMPRENANT

(30) Priority: 30.09.2020 US 202063085436 P
(43) Date of publication of application: 09.08.2023
(73) Proprietor: Koninklijke Philips N.V., 5656 AG Eindhoven (NL)
(72) Inventor: GRASHOW, Jonathan Sayer, 5656 AE Eindhoven (NL); MARGARIA, Elizabeth Powell, 5656 AE Eindhoven (NL); O'GRADY, Robert Edward, 5656 AE Eindhoven (NL)
(74) Representative: Philips Intellectual Property & Standards
(86) International application number: PCT/EP2021/076025
(87) International publication number: WO 2022/069309

(56) References cited:
- EP-A1- 2 420 281
- WO-A1-2016/119018
- WO-A1-2018/053589
- WO-A1-2018/085889
- WO-A1-2020/170207
- DE-A1- 102014 001 157
- US-A1- 2014 283 831

## Description

### CROSS-REFERENCE TO RELATED APPLICATIONS

This patent application claims the priority benefit under 35 U.S.C. § 119(e) of U.S. Provisional Application No. 63/085,436, filed on September 30, 2020.

### BACKGROUND OF THE INVENTION

### 1. Field of the Invention

The present invention pertains to patient interfaces for use in delivering a flow of a breathing gas to an airway of a patient. More particularly, the present invention pertains to exhaust arrangements for use in such patient interfaces. The present disclosure further pertains to a method of cleaning an exhaust arrangement utilized in patient interface.

### 2. Description of the Related Art

Many individuals suffer from disordered breathing during sleep. Sleep apnea is a common example of such sleep disordered breathing suffered by millions of people throughout the world. One type of sleep apnea is obstructive sleep apnea (OSA), which is a condition in which sleep is repeatedly interrupted by an inability to breathe due to an obstruction of the airway; typically the upper airway or pharyngeal area. Obstruction of the airway is generally believed to be due, at least in part, to a general relaxation of the muscles which stabilize the upper airway segment, thereby allowing the tissues to collapse the airway. Another type of sleep apnea syndrome is a central apnea, which is a cessation of respiration due to the absence of respiratory signals from the brain's respiratory center. An apnea condition, whether obstructive, central, or mixed, which is a combination of obstructive and central, is defined as the complete or near cessation of breathing, for example a 90% or greater reduction in peak respiratory airflow.

Those afflicted with sleep apnea experience sleep fragmentation and complete or nearly complete cessation of ventilation intermittently during sleep with potentially severe degrees of oxyhemoglobin desaturation. These symptoms may be translated clinically into extreme daytime sleepiness, cardiac arrhythmias, pulmonary-artery hypertension, congestive heart failure and/or cognitive dysfunction. Other consequences of sleep apnea include right ventricular dysfunction, carbon dioxide retention during wakefulness, as well as during sleep, and continuous reduced arterial oxygen tension. Sleep apnea sufferers may be at risk for excessive mortality from these factors as well as by an elevated risk for accidents while driving and/or operating potentially dangerous equipment.

Even if a patient does not suffer from a complete or nearly complete obstruction of the airway, it is also known that adverse effects, such as arousals from sleep, can occur where there is only a partial obstruction of the airway. Partial obstruction of the airway typically results in shallow breathing referred to as a hypopnea. A hypopnea is typically defined as a 50% or greater reduction in the peak respiratory airflow. Other types of sleep disordered breathing include, without limitation, upper airway resistance syndrome (UARS) and vibration of the airway, such as vibration of the pharyngeal wall, commonly referred to as snoring.

It is well known to treat sleep disordered breathing by applying a continuous positive air pressure (CPAP) to the patient's airway. This positive pressure effectively "splints" the airway, thereby maintaining an open passage to the lungs. It is also known to provide a positive pressure therapy in which the pressure of gas delivered to the patient varies with the patient's breathing cycle, or varies with the patient's breathing effort, to increase the comfort to the patient. This pressure support technique is referred to as bi-level pressure support, in which the inspiratory positive airway pressure (IPAP) delivered to the patient is higher than the expiratory positive airway pressure (EPAP). It is further known to provide a positive pressure therapy in which the pressure is automatically adjusted based on the detected conditions of the patient, such as whether the patient is experiencing an apnea and/or hypopnea. This pressure support technique is referred to as an auto-titration type of pressure support, because the pressure support device seeks to provide a pressure to the patient that is only as high as necessary to treat the disordered breathing.

Pressure support therapies as just described involve the placement of a patient interface including a mask component having a soft, flexible sealing cushion on the face of the patient. The mask component may be, without limitation, a nasal mask that covers the patient's nose, a nasal/oral mask that covers the patient's nose and mouth, or a full face mask that covers the patient's face. Such patient interfaces may also employ other patient contacting components, such as forehead supports, cheek pads and chin pads. The patient interface is typically secured to the patient's head by a headgear component. The patient interface is connected to a gas delivery tube or conduit and interfaces the pressure support device with the airway of the patient, so that a flow of breathing gas can be delivered from the pressure/flow generating device to the airway of the patient via the patient interface. An exhaust port (also referred to as an exhalation vent, exhalation port, and/or exhaust vent) is provided in the gas delivery tube or conduit and/or the patient interface device to allow exhaust gas, such as the exhaled gas from the patient, to vent to atmosphere.

Typical exhaust ports comprise a single orifice (e.g. a slot) or an array of smaller holes which extend directly from the interior of the patient interface and/or tube/conduit via the shortest path (i.e., generally perpendicular to the wall of the patient interface and/or tube/conduit) to the surrounding environment. Common complaints for such type of exhaust port are that the exhausted air blows onto the patient (e.g. hits the patient's hands) or a patient's bed partner resulting in an uncomfortable feeling. Some newer exhaust port designs have tried to reduce such air jetting by using a mesh material or by covering the exhaust port hole(s) with a fiber material to attempt to diffuse the exhausted air. A common complaint with such mesh/fiber approaches is that they are difficult for a patient to keep clean and may provide a place for germs to grow as they are difficult or impossible to clean.

WO 2018/085889 A1 discloses a gas washout vent for a patient interface which can be integrated within a plenum chamber or within an elbow connector. The vent comprises a housing with a first and a second wall, openings through the first wall to provide passages for fluid communication with a portion of the patient interface system, openings through the second wall to provide passages for fluid communication with ambient atmosphere, and a diffusing material located within the housing. The vent may comprise a deflector within the housing that prevents air flowing straight through the diffusing material. The deflector may be comprised of multiple pieces.

EP 2420281 A1 discloses a vent component for a mask comprising one or more tracks or grooves between the mask and the vent component adapted to guide gas washout.

US 2014/283831 A1 discloses a gas washout vent assembly which has a variable exhaust area.

WO 2018/053589 A1 discloses a gas washout vent having first openings and second openings, and wherein a diffusing material is positioned in the path between the first and second openings.

DE 102014001157 discloses vent housing 3401 for use during respiratory therapy with a flow of pressurised gas.

WO 2016/119018 A1 discloses a patient interface with an exhalation element which has air-permeable pores and may be formed of a fabric material.

### SUMMARY OF THE INVENTION

As one aspect of the present invention, there is provided an arrangement structured to provide for the passage of gases from a cavity of a patient interface to an ambient environment in which patient interface is disposed, the patient interface being for use in providing a flow of a treatment gas to the airway of a patient, according to claim 1. Preferred embodiments are matter of the dependent claims.

The one inlet may be in the shape of an elongated slot.

The number of high-drag passages may be defined by a corresponding number of grooves formed in the external member that are each bounded by a portion of the internal member thus defining each high-drag passage.

The number of grooves may be formed via one of: injection molding, machining, or 3D printing.

The external member may be selectively coupled to the internal member.

The internal member may comprise a portion of the patient interface.

Each high-drag passage may comprise at least one of: a number of textured surfaces, multiple bends, a length of at least 20mm, and/or a minor diameter of 0.8 mm or less.

As another aspect of the present invention, there is provided a patient interface for use in providing a flow of a treatment gas to the airway of a patient according to claim 8.

According to the invention, the body member of the arrangement comprises an internal member and an external member coupled to the internal member. According to an exemplary embodiment, a number of inlets are defined in the internal member, and the number of outlets are defined in the external member.

Each high-drag passage may comprise at least one of: a number of textured surfaces, multiple bends, a length of at least 20mm, and/or a minor diameter of 0.8 mm or less.

As yet a further aspect, there is provided a respiratory interface system for use in providing a regimen of respiratory therapy to a patient according to claim 9.

Each high-drag passage may comprise at least one of: a number of textured surfaces, multiple bends, a length of at least 20mm, and/or a minor diameter of 0.8 mm or less.

These and other objects, features, and characteristics of the present invention, as well as the methods of operation and functions of the related elements of structure and the combination of parts and economies of manufacture, will become more apparent upon consideration of the following description and the appended claims with reference to the accompanying drawings, all of which form a part of this specification, wherein like reference numerals designate corresponding parts in the various figures. It is to be expressly understood, however, that the drawings are for the purpose of illustration and description only and are not intended as a definition of the limits of the invention.

### BRIEF DESCRIPTION OF THE DRAWINGS

FIG. 1 is a partially schematic depiction of a respiratory interface system for use in providing a flow of positive pressure breathing gas to the airway of a patient including an arrangement for providing for the passage of gases from a cavity of the patient interface in accordance with one example embodiment of the present invention, shown with a patient interface thereof disposed on the head of a patient;
FIG. 2 is a front elevation view of the patient interface of FIG. 1;
FIG. 3 is a section view of the patient interface of FIGS. 1 and 2 taken along line 3-3 of FIG. 2;
FIG. 4 is a perspective view of an inlet side of another arrangement for providing for the passage of gases from a cavity of a patient interface to an ambient environment in accordance with one example embodiment of the present invention;
FIG. 5 is a perspective view of the opposite, outlet side of the arrangement of FIG. 4;
FIG. 6 is an exploded view of the arrangement of FIGS. 4 and 5;
FIG. 7 is a perspective view of an internal face of an external member of the arrangement of FIGS. 4-6;
FIG. 8 is another perspective view of the inlet side of the arrangement of FIGS. 4-6 shown generally transparent in order to show details of internal structures thereof;
FIG. 9 is a partially schematic depiction of another respiratory interface system for use in providing a flow of positive pressure breathing gas to the airway of a patient having a patient interface, shown in front elevation, including another arrangement for providing for the passage of gases from a cavity of the patient interface in accordance with one example embodiment of the present invention;
FIG. 10 is a rear elevation view of the patient interface of FIG. 9;
FIG. 11 is a front perspective view of the patient interface of FIGS. 9 and 10;
FIG. 12 is a front exploded perspective view of the patient interface of FIGS. 9-11; and
FIG. 13 is a rear exploded perspective view of the patient interface of FIGS. 9-11.

### DETAILED DESCRIPTION OF EXEMPLARY EMBODIMENTS

As used herein, the singular form of "a", "an", and "the" include plural references unless the context clearly dictates otherwise. As used herein, the statement that two or more parts or components are "coupled" shall mean that the parts are joined or operate together either directly or indirectly, i.e., through one or more intermediate parts or components, so long as a link occurs. As used herein, "directly coupled" means that two elements are directly in contact with each other. As used herein, "fixedly coupled" or "fixed" means that two components are coupled so as to move as one while maintaining a constant orientation relative to each other.

Directional phrases used herein, such as, for example and without limitation, top, bottom, left, right, upper, lower, front, back, and derivatives thereof, relate to the orientation of the elements shown in the drawings and are not limiting upon the claims unless expressly recited therein.

As used herein, the statement that two or more parts or components "engage" one another shall means that the parts exert a force against one another either directly or through one or more intermediate parts or components.

As used herein, the word "unitary" means a component is created as a single piece or unit. That is, a component that includes pieces that are created separately and then coupled together as a unit is not a "unitary" component or body.

As used herein, the term "number" shall mean one or an integer greater than one (i.e., a plurality).

As used herein, a "coupling assembly" includes two or more couplings or coupling components. The components of a coupling or coupling assembly are generally not part of the same element or other component. As such the components of a "coupling assembly" may not be described at the same time in the following description.

As used herein, a "coupling" is one element of a coupling assembly. That is, a coupling assembly includes at least two components, or coupling components, that are structured to be coupled together. It is understood that the elements of a coupling assembly are compatible with each other. For example, in a coupling assembly, if one coupling element is a snap socket, the other coupling element is a snap plug.

As used herein, "correspond" indicates that two structural components are sized and shaped to be similar to each other and may be coupled with a minimum amount of friction. Thus, an opening which "corresponds" to a member is sized slightly larger than the member so that the member may pass through the opening with a minimum amount of friction. This definition is modified if the two components are said to fit "snugly" together or "snuggly correspond." In that situation, the difference between the size of the components is even smaller whereby the amount of friction increases. If the element defining the opening and/or the component inserted into the opening is/are made from a deformable or compressible material, the opening may even be slightly smaller than the component being inserted into the opening. This definition is further modified if the two components are said to "substantially correspond." "Substantially correspond" means that the size of the opening is very close to the size of the element inserted therein. That is, not so close as to cause substantial friction, as with a snug fit, but with more contact and friction than a "corresponding fit," i.e. a "slightly larger" fit.

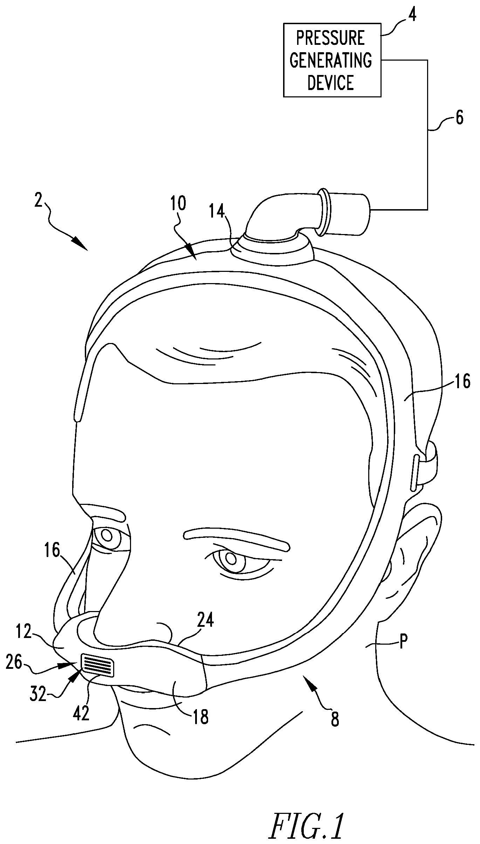

A respiratory interface system 2 adapted to provide a regimen of respiratory therapy to a patient P according to one exemplary embodiment of the present invention is shown in FIG. 1. Respiratory interface system 2 includes a pressure generating device 4 (shown schematically), and a delivery conduit 6 (shown schematically) fluidly coupled to a mask 8. Pressure generating device 4 is structured to generate a flow of positive pressure breathing gas and may include, without limitation, ventilators, constant pressure support devices (such as a continuous positive airway pressure device, or CPAP device), variable pressure devices (e.g., BiPAP^{®}, Bi-Flex^{®}, or C-Flex^{™} devices manufactured and distributed by Philips Respironics of Murrysville, PA), and auto-titration pressure support devices. Delivery conduit 6 is structured to communicate the flow of breathing gas from pressure generating device 4 to mask 8, and mask 8 is structured to further communicate the flow of breathing gas received from conduit 6 to an airway of patient P. Delivery conduit 6 and mask 8 are often collectively referred to as a patient circuit.

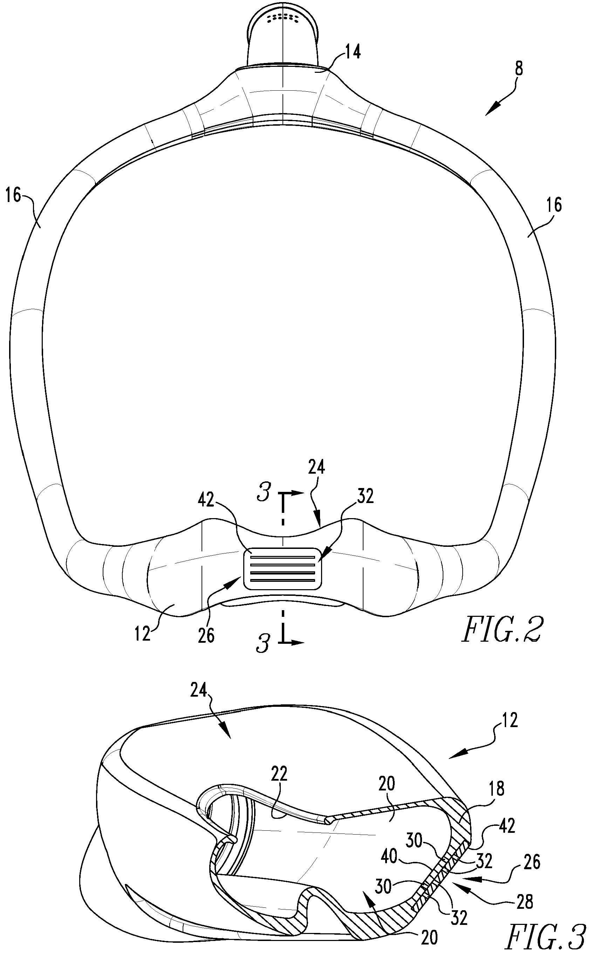

Continuing to refer to FIG. 1, as well as to FIG. 2, mask 8 includes a tubing assembly 10 and a patient interface 12 coupled to tubing assembly 10. Tubing assembly 10 includes a manifold portion 14 structured to receive the flow of positive pressure breathing gas from delivery conduit 6, a number (two are shown in the example of FIGS. 1 and 2) of tubular portions 16 which each extend from manifold portion 14 to a distal end (not numbered) which is selectively coupled to patient interface 12. It is to be appreciated that other arrangements aside from tubing assembly 10 may be employed in mask 8 to provide the flow of positive pressure breathing gas produced by pressure generating device 4 to patient interface 12 without varying from the scope of the present invention.

Referring now to FIG. 3, in addition to FIGS. 1 and 2, patient interface 12 includes a body 18 that defines a cavity 20 therein that is structured to receive (e.g., via tubing assembly 10) the flow of positive pressure breathing gas produced by pressure generating device 4. In the one example shown in FIGS. 1-3, body 18 is made of a soft, flexible material, such as, without limitation, silicone, an appropriately soft thermoplastic elastomer, a closed-cell foam, or any other suitable material or combination of such materials, however, it is to be appreciated that body 18 may be formed from one or more other suitable materials without varying from the scope of the present invention. Patient interface 12 further includes a first aperture 22 defined therein that is positioned and structured to communicate the flow of breathing gas from cavity 20 to the airway of the patient and a sealing element 24 disposed thereabout first aperture 22 which is structured to sealingly engage about one or more of the nares and/or mouth of patient P (depending on the particular patient interface arrangement).

In the example embodiment illustrated in FIGS. 1-3, sealing element 24 is formed as an integral portion of body 18, and thus is made of a soft, flexible material, such as, without limitation, silicone, an appropriately soft thermoplastic elastomer, a closed-cell foam, or any other suitable material or combination of such materials. It is to be appreciated, however, that sealing element 24 made be formed as a separate element and may take the form of any type of patient sealing element, such as a nasal/oral mask, a nasal pillow or a full face mask, which facilitates the delivery of the flow of breathing gas to the airway of a patient, without varying from the scope of the present invention. Patient interface 12 further includes an arrangement 26 coupled to, or provided in-part as a portion of, body 18 that provides for the passage of gases (e.g., patient exhalation gases) from cavity 20 of patient interface 12 to an ambient environment in which body 18, and thus patient interface 12, is disposed.

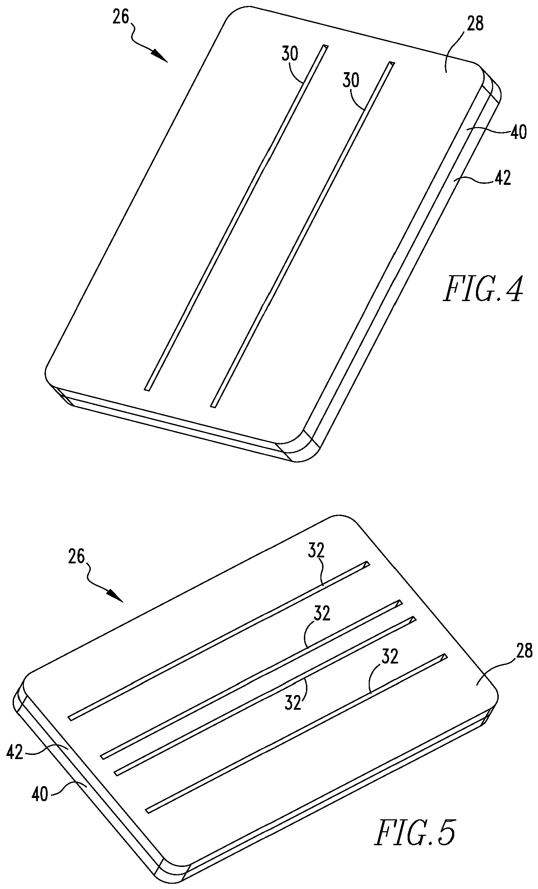

Referring now to FIGS. 4-8, in addition to FIGS. 1-3, arrangement 26 includes a generally thin, plate-like body member 28 formed from a plastic or other suitable rigid or semi-rigid material (e.g., metal, elastomer, etc.) that is sized and configured to form a portion of patient interface 12 between cavity 20 and the ambient environment in which the patient interface is disposed. Body member 28 includes a number of inlets 30 defined in an inlet side thereof, with each inlet 30 being positioned and structured to receive gases from cavity 20. In the example shown in FIGS. 4-8, two inlets 30 generally shaped as elongated slots are provided, however, it is to be appreciated that either or both of the quantity and/or shape of number inlets 30 may be varied without varying form the scope of the present invention. Body member 28 further includes a number of outlets 32 defined in an outlet side thereof, opposite the inlet side, with each outlet 32 being positioned and structured to provide for the exit of gases from arrangement 26, and thus patient interface 12, to the ambient environment. In the example shown in FIGS. 4-8, four outlets 32 generally shaped as elongated slots are provided, however, it is to be appreciated that either or both of the quantity and/or shape of number outlets 32 may be varied without varying form the scope of the present invention.

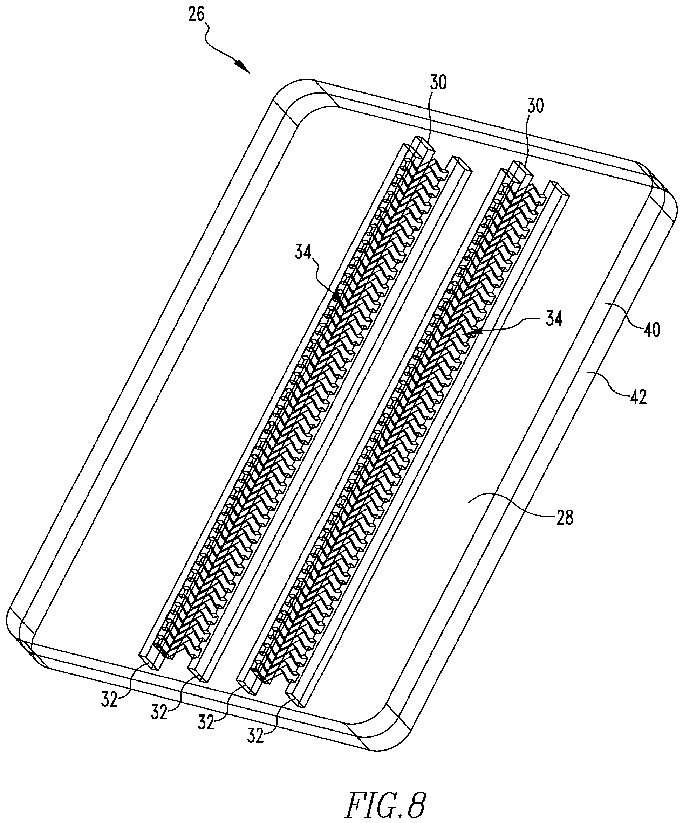

Body member 28 also includes a number of high-drag passages 34 defined therein, with each high-drag passage extending laterally (i.e., within body member 28 generally parallel to outer surfaces thereof and/or perpendicular to a straight through passage) a distance between an inlet 30 of the number of inlets 30 and an outlet 32 of the number of outlets 32. As used herein, a "high drag passage" is a passage having walls dimensioned and/or positioned so as to slow the airflow velocity of gases passing therethrough without the use of a blocking material positioned therein. For example, a passage having a fibrous material disposed therein is not a "high-drag passage". As another example, a passage extending straight through a wall (i.e., perpendicular to the wall) is also not a "high drag passage". In the example shown in FIGS. 4-8, each high-drag passage includes opposing sidewalls having 90 degree bends that act to slow the airflow velocity of gases passing therethrough. Other arrangements of high-drag passages may include, for example, without limitation, one or more of: textured surfaces, multiple bends, long/narrow passages (e.g., passages having a length of 20mm or more, passages having a minor diameter (i.e., the smaller dimension of an elliptical or rectangular channel cross-section) of 0.2mm to 8mm. The example shown in FIGS. 4-8 includes a plurality of high-drag passages 34 (FIG. 8) extending between each inlet 30 and outlet 32. However, it is to be appreciated that the quantity of high-drag passages and/or the quantity of inlets and/or outlets in which they extend between may be varied without varying from the scope of the present invention.

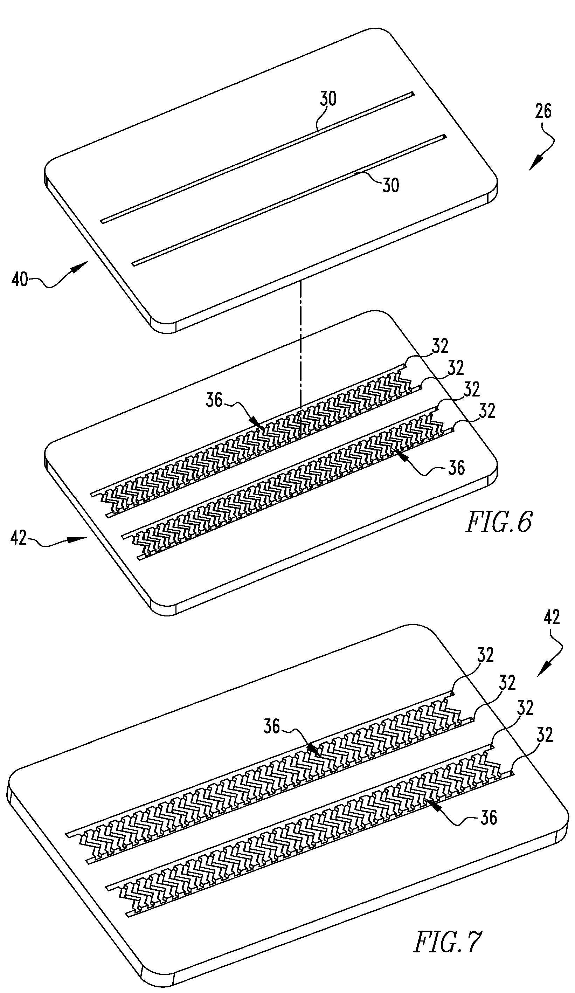

As shown in the example embodiment of FIGS. 4-8, body member 28 may be formed from a generally thin, plate-like, internal member 40 structured to be positioned bounding cavity 20, and a generally thin, plate-like, external member 42 that is coupled to internal member 40 and positioned to be in communication with the ambient environment in which patient interface 12 is disposed. In such example embodiment the number of inlets 30 are defined in, and through internal member 40, while the number of outlets 32 are defined in and through external member. Also in such embodiment the number of high-drag passages 34 are defined in-part by a corresponding number of channels or grooves 36 (FIGS. 6 and 7) defined in external member 42 and, when external member 42 is coupled to internal member 40, by corresponding portions of internal member 40. Alternatively, the number of high-drag passages 34 may be similarly formed by channels or grooves defined in internal member 40 that interact with corresponding portions of external member 42 or by channels or grooves formed in both internal member 40 and external member 42 that interact with corresponding portions of the other member 40, 42 to define high-drag passages 34. As shown in the sectional view of FIG. 3, internal member 40 may be coupled to, or formed as an integral portion of, body 18 of patient interface 12. Alternatively, external member 42 may be coupled to, or formed as an integral portion of, body 18 of patient interface 12.

By relying on the geometry of mating surfaces of multiple components to create the enclosed high-drag passages 34, it is possible to create passages that otherwise could not be easily manufactured due to their small size, small diameter to length ratio, and/or torturous shape. The channels or grooves from which the high-drag passages are defined in-part may be formed from any suitable method such as, for example, without limitation, injection molding, machining, chemical etching, and/or 3D printing. In addition to providing for forming passages of complex geometries to be readily created, such modular construction additionally provides for the elements to be separated without damage if/when selectively coupled together (e.g., a snap fit, interference fit, or other suitable arrangement), thus exposing the channels that form each of the high-drag passages for cleaning thereof and/or replacement depending on the particular application.

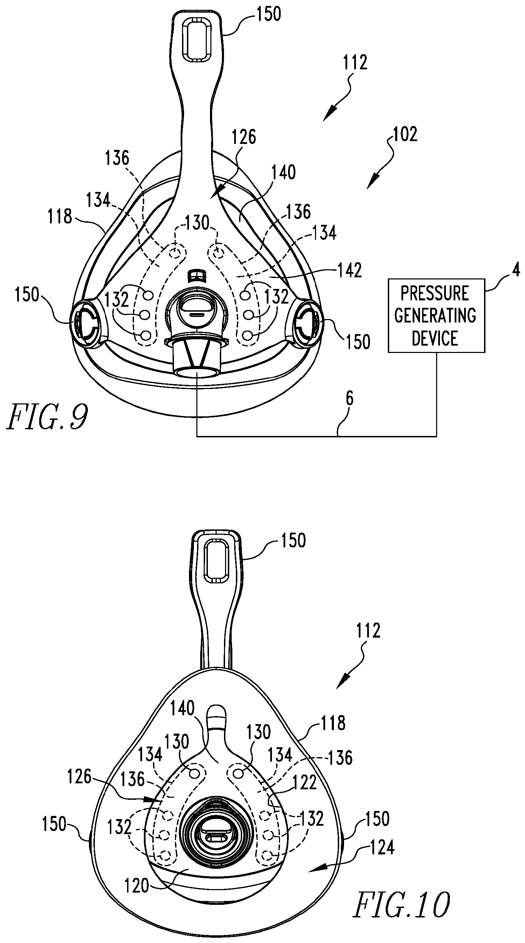

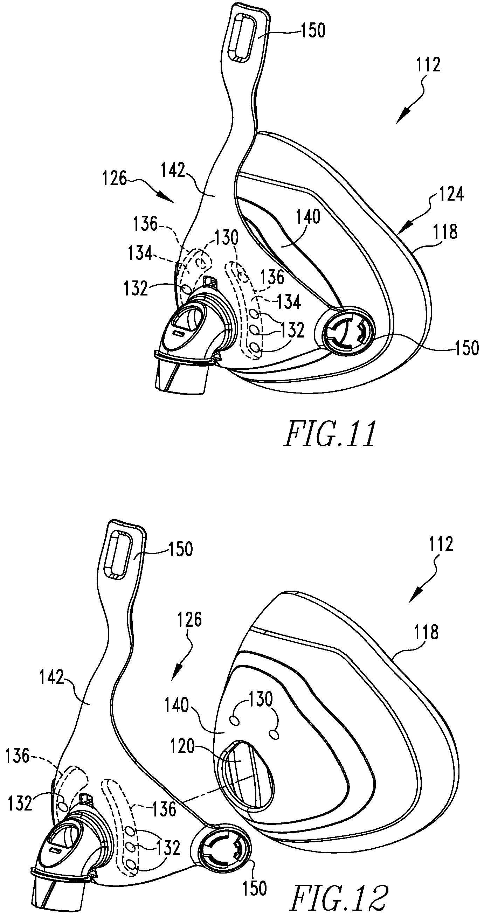

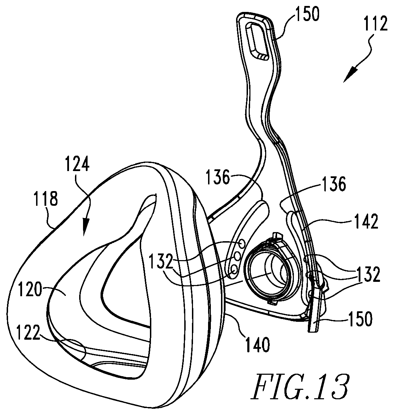

Another respiratory interface system 102 adapted to provide a regimen of respiratory therapy to a patient P according to another exemplary embodiment of the present invention is shown in FIG. 9. Like respiratory interface system 2 previously discussed, respiratory interface system 102 includes pressure generating device 4 (shown schematically) coupled, via conduit 6 (also shown schematically), to a patient interface 112 for providing the flow of positive pressure breathing gas thereto. Continuing to refer to FIG. 9, as well as to FIGS. 10-13, patient interface 112 includes a body 118 that defines a cavity 120 therein that is structured to receive (e.g., via conduit 6) the flow of positive pressure breathing gas produced by pressure generating device 4. In the one example shown in FIGS. 9-13, body 118 is made of a soft, flexible material, such as, without limitation, silicone, an appropriately soft thermoplastic elastomer, a closed-cell foam, or any other suitable material or combination of such materials, however, it is to be appreciated that body 118 may be formed from one or more other suitable materials without varying from the scope of the present invention. Patient interface 112 further includes a first aperture 122 defined therein that is positioned and structured to communicate the flow of breathing gas from cavity 120 to the airway of the patient and a sealing element 124 disposed thereabout first aperture 122 which is structured to sealingly engage about one or more of the nares and/or mouth of patient P (depending on the particular patient interface arrangement).

Patient interface 112 further includes an arrangement 126 coupled to, or provided in-part as a portion of, body 118 that provides for the passage of gases (e.g., patient exhalation gases) from cavity 120 of patient interface 12 to an ambient environment in which body 118, and thus patient interface 112, is disposed. Similar to arrangement 26 previously discussed, arrangement 126 includes a generally thin, plate-like, internal member in the form of a generally rigid faceplate 140 that is positioned bounding cavity 120 and generally surrounded by sealing element 124, and a generally thin, plate-like, external member in the form of a frame 142 that may be permanently or selectively coupled to faceplate 140 and positioned in communication with the ambient environment in which patient interface 112 is disposed. Frame 142 includes a number of securement points or structures 150 to which a suitable headgear or other stabilizing (e.g., a forehead support) and/or securement arrangement (not shown) may be coupled in order to secure/stabilize patient interface on the face/head of a patient. In such example embodiment, a number of inlets 130 are defined in, and through faceplate 140, while a number of outlets 132 (shown generally schematically enlarged in order to merely demonstrate their general positioning in the example embodiment) are defined in and through frame 142. Additionally in such embodiment, a number of high-drag passages 134 are defined in-part by a corresponding number of channels or grooves 136 defined in frame 142 and, when frame 142 is coupled to faceplate 140, by corresponding portions of faceplate 140. Alternatively, the number of high-drag passages 134 may be similarly formed by channels or grooves defined in faceplate 140 that interact with corresponding portions of frame 142 or by channels or grooves formed in both faceplate 140 and frame 142 that interact with corresponding portions of the other element 140, 142 to define high-drag passages 134.

The example arrangement 126 shown in FIGS. 9-13 includes high-drag passages 134, each with one circular inlet 130 and three circular outlets 132 associated therewith. It is to be appreciated, however, that such particular arrangement is shown for exemplary purposes only and that one or more of: the quantity, sizing, and/or layout of such high-drag passages 134; the quantity, sizing, and/or shape of the inlets 130; and the quantity, sizing, and/or shape of the outlets 132 may be varied without varying from the scope of the present invention.

From the foregoing examples it is to be appreciated that embodiments of the present invention provide arrangements for exhausting gases from a patient interface that improve upon known solutions. Such arrangements may include anywhere form a single large channel to an array of hundreds of channels depending on the particular application. When more channels are utilized, channels of shorter length and/or lesser cross-sectional area are needed. Accordingly, embodiments of the present invention can generally be tailored as needed to fit a particular patient interface arrangement.

Although the invention has been described in detail for the purpose of illustration based on what is currently considered to be the most practical and preferred embodiments, it is to be understood that such detail is solely for that purpose and that the invention is not limited to the disclosed embodiments, but, on the contrary, is intended to cover modifications that are within the scope of the appended claims. For example, it is to be understood that the present invention contemplates that, to the extent possible, one or more features of any embodiment can be combined with one or more features of any other embodiment.

In the claims, any reference signs placed between parentheses shall not be construed as limiting the claim. The word "comprising" or "including" does not exclude the presence of elements or steps other than those listed in a claim. In a device claim enumerating several means, several of these means may be embodied by one and the same item of hardware. The word "a" or "an" preceding an element does not exclude the presence of a plurality of such elements. In any device claim enumerating several means, several of these means may be embodied by one and the same item of hardware. The mere fact that certain elements are recited in mutually different dependent claims does not indicate that these elements cannot be used in combination.

## Claims

1. An arrangement (26) structured to provide for the passage of gases from a cavity (20) of a patient interface (12) to an ambient environment in which the patient interface is disposed, the patient interface being for use in providing a flow of a treatment gas to the airway of a patient, the arrangement comprising:
a body member (18) sized and configured to form a portion of the patient interface between the cavity and the ambient environment, the body member comprising:
an inlet (30) defined in a first side of the body member, the inlet being positioned and structured to receive gases from the cavity;
a pair of outlets (32) defined in a second side of the body member opposite the first side, each outlet being positioned and structured to provide for the exit of gases from the patient interface to the ambient environment, wherein the inlet is disposed between the pair of outlets; and
a plurality of high-drag passages (34) defined in the body member, each passage communicating with the inlet at a central portion of the passage and extending between the pair of outlets,
wherein the body member comprises an internal member (40) and an external member (42) coupled to the internal member, wherein the inlet (30) is defined in the internal member (40), and wherein the pair of outlets (32) are defined in the external member (42),
wherein the plurality of high-drag passages (34) are defined by a corresponding number of grooves (36) formed in one or both of the internal member (40) and/or the external member (42), and
wherein the number of high-drag passages (34) defines a tortuous path between the inlet and each outlet in the pair of outlets.

2. The arrangement of claim 1, wherein the inlet is in the shape of an elongated slot.

3. The arrangement of claim 1, wherein the plurality of passages are defined by a corresponding number of grooves (36) formed in the external member that are each bounded by a portion of the internal member thus defining each passage.

4. The arrangement of claim 3, wherein the number of grooves are formed via one of: injection molding, machining, etching, or 3D printing.

5. The arrangement of claim 1, wherein the external member is selectively coupled to the internal member.

6. The arrangement of claim 1, wherein the internal member comprises a portion of the patient interface.

7. The arrangement of claim 1, wherein each passage comprises at least one of: a number of textured surfaces, multiple bends, a length of at least 20mm, and/or a minor diameter of 0.8 mm or less.

8. A patient interface (12) for use in providing a flow of a treatment gas to the airway of a patient, the patient interface comprising:
(a) a body (18) that defines a cavity (20) therein that is structured to receive the flow of treatment gas;
(b) a first aperture (22) defined therein that is positioned and structured to communicate the flow of treatment gas from the cavity to the airway of the patient;
(c) a sealing element (24) disposed thereabout the first aperture, the sealing element being structured to sealingly engage about one or more of the nares and/or mouth of the patient; and
(d) an arrangement (26) in accordance with any of claims 1-7, structured to provide for the passage of gases from the cavity to an ambient environment in which the patient interface is disposed.

9. A respiratory interface system (2) for use in providing a regimen of respiratory therapy to a patient, the system comprising:
(a) a pressure generating device (4) structured to provide a flow of a treatment gas;
(b) a patient interface (12) in accordance with claim 8, structured communicate the flow of treatment gas to an airway of the patient; and
(c) a delivery conduit (6) coupled between the pressure generating device and the patient interface, the delivery conduit structured to communicate the flow of the treatment gas from the pressure generating device to the patient interface.

## Patentansprüche

1. Anordnung (26), die strukturiert ist, um den Durchgang von Gasen aus einem Hohlraum (20) einer Patientenschnittstelle (12) in eine umliegende Umgebung bereitzustellen, in der die Patientenschnittstelle angeordnet ist, wobei die Patientenschnittstelle zur Verwendung beim Bereitstellen eines Flusses eines Behandlungsgases in die Atemwege eines Patienten dient, wobei die Anordnung umfasst:
ein Körperelement (18), das bemessen und konfiguriert ist, um einen Abschnitt der Patientenschnittstelle zwischen dem Hohlraum und der umliegenden Umgebung zu bilden, wobei das Körperelement umfasst:
einen Einlass (30), der auf einer ersten Seite des Körperelements definiert ist, wobei der Einlass positioniert und strukturiert ist, um Gase aus dem Hohlraum aufzunehmen;
ein Paar von Auslässen (32), die auf einer zweiten Seite des Körperelements gegenüber der ersten Seite definiert sind, wobei jeder Auslass positioniert und strukturiert ist, um den Austritt von Gasen aus der Patientenschnittstelle in die umliegende Umgebung bereitzustellen, wobei der Einlass zwischen dem Paar von Auslässen angeordnet ist; und
eine Vielzahl von im Körperelement definierten Durchgängen (34) mit hohem Widerstand, wobei jeder Durchgang an einem zentralen Abschnitt des Durchgangs mit dem Einlass in Verbindung steht und sich zwischen dem Paar von Auslässen erstreckt,
wobei das Körperelement ein inneres Element (40) und ein mit dem inneren Element gekoppeltes äußeres Element (42) umfasst, wobei der Einlass (30) in dem inneren Element (40) definiert ist, und wobei das Paar von Auslässen (32) in dem äußeren Element (42) definiert ist,
wobei die Vielzahl von Durchgängen (34) mit hohem Widerstand durch eine entsprechende Anzahl von Nuten (36) definiert sind, die in einem oder beiden von dem inneren Element (40) und/oder dem äußeren Element (42) gebildet sind, und
wobei die Anzahl von Durchgängen (34) mit hohem Widerstand einen gewundenen Weg zwischen dem Einlass und jedem Auslass in dem Paar von Auslässen definiert.

2. Anordnung nach Anspruch 1, wobei der Einlass in der Form eines länglichen Schlitzes ist.

3. Anordnung nach Anspruch 1, wobei die Vielzahl von Durchgängen durch eine entsprechende Anzahl von Nuten (36) definiert sind, die in dem äußeren Element gebildet sind, die jeweils durch einen Abschnitt des inneren Elements begrenzt sind, wodurch jeder Durchgang definiert wird.

4. Anordnung nach Anspruch 3, wobei die Anzahl von Nuten durch eines von Spritzguss, Bearbeiten, Ätzen oder 3D-Druck gebildet wird.

5. Anordnung nach Anspruch 1, wobei das äußere Element selektiv mit dem inneren Element gekoppelt ist.

6. Anordnung nach Anspruch 1, wobei das innere Element einen Abschnitt der Patientenschnittstelle umfasst.

7. Anordnung nach Anspruch 1, wobei jeder Durchgang mindestens eines von einer Anzahl texturierter Oberflächen, mehreren Biegungen, einer Länge von mindestens 20 mm, und/oder einem Kerndurchmesser von 0,8 mm oder weniger umfasst.

8. Patientenschnittstelle (12) zur Verwendung beim Bereitstellen eines Flusses von Behandlungsgas in die Atemwege eines Patienten, wobei die Patientenschnittstelle umfasst:
(a) einen Körper (18), der einen Hohlraum (20) darin definiert, der strukturiert ist, um den Fluss von Behandlungsgas aufzunehmen;
(b) eine darin definierte erste Öffnung (22), die positioniert und strukturiert ist, um den Fluss von Behandlungsgas von dem Hohlraum in die Atemwege des Patienten zu kommunizieren;
(c) ein Dichtungselement (24), das um die erste Öffnung herum angeordnet ist, wobei das Dichtungselement strukturiert ist, um dichtend um eines oder mehr der Nasenlöcher und/oder Mund des Patienten herum einzugreifen; und
(d) eine Anordnung (26) nach einem der Ansprüche 1-7, die strukturiert ist, um den Durchgang von Gasen aus dem Hohlraum in eine umliegende Umgebung bereitzustellen, in der die Patientenschnittstelle angeordnet ist.

9. Beatmungsanschlusssystem (2) zur Verwendung beim Bereitstellen eines Atmungstherapieplans für einen Patienten, wobei das System umfasst:
(a) eine Druckerzeugungsvorrichtung (4), die strukturiert ist, um einen Fluss von Behandlungsgas bereitzustellen;
(b) eine Patientenschnittstelle (12) nach Anspruch 8, die strukturiert ist, um den Fluss von Behandlungsgas in die Atemwege des Patienten zu kommunizieren; und
(c) eine Abgabeleitung (6), die zwischen der Druckerzeugungsvorrichtung und der Patientenschnittstelle gekoppelt ist, wobei die Abgabeleitung strukturiert ist, um den Fluss des Behandlungsgases von der Druckerzeugungsvorrichtung zu der Patientenschnittstelle zu kommunizieren.

## Revendications

1. Agencement (26) structuré pour permettre le passage de gaz depuis une cavité (20) d'une interface patient (12) vers un environnement ambiant, dans lequel est disposée l'interface patient, l'interface patient étant destinée à être utilisée pour fournir un flux de gaz de traitement aux voies respiratoires d'un patient, l'agencement comprenant :
un élément de corps (18) dimensionné et configuré pour former une partie de l'interface patient entre la cavité et l'environnement ambiant, l'élément de corps comprenant :
une entrée (30) définie dans un premier côté de l'élément de corps, l'entrée étant positionnée et structurée pour recevoir des gaz de la cavité ;
une paire de sorties (32) définies dans un second côté de l'élément de corps opposé au premier côté, chaque sortie étant positionnée et structurée pour permettre la sortie de gaz de l'interface patient vers l'environnement ambiant, dans lequel l'entrée est disposée entre la paire de sorties ; et
une pluralité de passages à forte résistance au courant (34) définis dans l'élément de corps, chaque passage communiquant avec l'entrée au niveau d'une partie centrale du passage et s'étendant entre la paire de sorties,
dans lequel l'élément de corps comprend un élément interne (40) et un élément externe (42) couplé à l'élément interne, dans lequel l'entrée (30) est définie dans l'élément interne (40), et dans lequel la paire de sorties (32) est définie dans l'élément externe (42),
dans lequel la pluralité de passages à forte résistance au courant (34) sont définis par un nombre correspondant de rainures (36) formées dans l'un ou les deux de l'élément interne (40) et/ou de l'élément externe (42), et
dans lequel le nombre de passages à forte résistance au courant (34) définit un chemin sinueux entre l'entrée et chaque sortie dans la paire de sorties.

2. Agencement selon la revendication 1, dans lequel l'entrée présente la forme d'une fente allongée.

3. Agencement selon la revendication 1, dans lequel la pluralité de passages est définie par un nombre correspondant de rainures (36) formées dans l'élément externe qui sont chacune délimitées par une partie de l'élément interne définissant ainsi chaque passage.

4. Agencement selon la revendication 3, dans lequel le nombre de rainures est formé par l'une des méthodes suivantes : moulage par injection, usinage, gravure ou impression 3D.

5. Agencement selon la revendication 1, dans lequel l'élément externe est sélectivement couplé à l'élément interne.

6. Agencement selon la revendication 1, dans lequel l'élément interne comprend une partie de l'interface patient.

7. Agencement selon la revendication 1, dans lequel chaque passage comprend au moins un parmi : un certain nombre de surfaces texturées, de multiples courbures, une longueur d'au moins 20 mm et/ou un diamètre mineur de 0,8 mm ou moins.

8. Interface patient (12) destinée à être utilisée pour fournir un flux d'un gaz de traitement aux voies respiratoires d'un patient, l'interface patient comprenant :
(a) un corps (18) qui définit une cavité (20) à l'intérieur qui est structurée pour recevoir le flux de gaz de traitement ;
(b) une première ouverture (22) définie à l'intérieur qui est positionnée et structurée pour communiquer le flux de gaz de traitement de la cavité aux voies respiratoires du patient ;
(c) un élément d'étanchéité (24) disposé autour de la première ouverture, l'élément d'étanchéité étant structuré pour se mettre en prise de manière étanche autour d'une ou plusieurs des narines et/ou de la bouche du patient ; et
(d) un agencement (26) selon l'une quelconque des revendications 1-7, structuré pour permettre le passage de gaz de la cavité vers un environnement ambiant dans lequel l'interface patient est disposée.

9. Système d'interface respiratoire (2) destiné à être utilisé pour fournir un régime de thérapie respiratoire à un patient, le système comprenant :
(a) un dispositif de génération de pression (4) structuré pour fournir un flux de gaz de traitement ;
(b) une interface patient (12) selon la revendication 8, structurée pour communiquer le flux de gaz de traitement à une voie respiratoire du patient ; et
(c) un conduit d'alimentation (6) couplé entre le dispositif de génération de pression et l'interface patient, le conduit d'alimentation étant structuré pour communiquer le flux de gaz de traitement depuis le dispositif de génération de pression jusqu'à l'interface patient.
